# EUROPÄISCHE PATENTSCHRIFT

(11) **EP 2 276 433 B1**
(45) Veröffentlichungstag und Bekanntmachung des Hinweises auf die Patenterteilung: **23.11.2011**
(21) Anmeldenummer: 09749498.3
(22) Anmeldetag: 20.05.2009
(51) Int. Cl.: A61F 5/01

(54) **KNIEORTHESE**
KNEE ORTHOSIS
ORTHÈSE DE GENOU

(30) Priorität: 20.05.2008 DE 102008024748
(43) Veröffentlichungstag der Anmeldung: 26.01.2011
(60) Teilanmeldung: 11004660.4
(73) Patentinhaber: Otto Bock HealthCare GmbH, 37115 Duderstadt (DE)
(72) Erfinder: AUBERGER, Roland, A-1020 Wien (AT)
(74) Vertreter: Stornebel, Kai
(86) Internationale Anmeldenummer: PCT/DE2009/000734
(87) Internationale Veröffentlichungsnummer: WO 2009/140956

(56) Entgegenhaltungen:
- WO-A-01/43669
- DE-A1- 3 911 780
- US-A- 5 252 102
- US-A- 5 547 464
- US-B1- 6 377 178

## Beschreibung

Die Erfindung betrifft eine Knieorthese mit einer Oberschenkelstruktur, insbesondere Oberschenkelschiene, die eine Befestigungseinrichtung zur Festlegung an einem Oberschenkel aufweist, einer Unterschenkelstruktur, insbesondere Unterschenkelschiene, die über eine Gelenkeinrichtung mit der Oberschenkelstrukur verschwenkbar gekoppelt ist und eine Befestigungseinrichtung zur Festlegung an einem Unterschenkel sowie ggf. ein Fußteil zur Abstützung eines Fußes aufweist, und einer Aktuatoreinheit zwischen der Oberschenkelstruktur und der Unterschenkelstruktur.

Knieorthesen werden zur Unterstützung oder zum Ersetzen einer Funktion eines Beines eingesetzt. Die Orthesen bilden einen äußeren Rahmen oder Teilrahmen und werden an dem Bein angelegt. Bei Verletzungen der Bänder oder der Muskulatur sowie Lähmungen dienen Orthesen zu einer Gelenkstabilisierung sowie ggf. zu einer Begrenzung des Beugewinkels oder Streckwinkels der über das Kniegelenk verbundenen Schenkelteile. In der Regel weisen die Knieorthesen je eine Schiene für den Oberschenkel und den Unterschenkel auf und werden über Befestigungseirichtungen, wie Schnallen oder Riemen, an dem Ober- und Unterschenkel festgelegt.

Bei Lähmungen des Beines oder bei entsprechenden Verletzungen ist es vorgesehen, dass auch der Fuß innerhalb der Orthese fixiert wird. Dazu ist eine Fußschale vorgesehen, die an der Unterschenkelstruktur befestigt oder daran ausgebildet ist.

Die DE 601 22 483 T2 beschreibt eine dynamische, elektromechanische, orthetische Vorrichtung mit einer Schlingfederkupplung, die auf Grundlage von Daten von Sensoren, die in dem Plantarbereich der Fußschale angeordnet sind, die Gelenkeinrichtung zwischen dem Oberschenkelteil und dem Unterschenkelteil freigibt. Ebenfalls ist ein kinematischer Sensor vorgesehen, der ein elektrisches Signal erzeugt, das aufgrund der relativen Position und Bewegung der Oberschenkelstruktur zu der Unterschenkelstruktur ermittelt wird. Über dieses Signal wird gesteuert, ob eine Schlingfeder aktuiert oder freigegeben wird.

Die DE 10 2006 012 716 A1 beschreibt eine Gelenkeinrichtung, mit deren Hilfe eine Bewegung um eine Achse freigegeben oder gesperrt wird. Es ist eine Sensoreinrichtung vorgesehen, die Kräfte, Momente und/oder Winkelstellung der Ober- und Unterteile zueinander misst und mit einer Steuereinheit gekoppelt ist, die in Abhängigkeit von den gemessenen Größen einen Aktuator aktiviert und eine Spiralfeder verspannt, um das Gelenk zu sperren oder freizugeben.

Die DE 202 17 355 U1 beschreibt eine Orthese zur äußeren Abstützung und Führung eines Kniegelenkes mit einem Fersen- und Fußteil, das an einer Unterschenkelstruktur angeordnet ist, die über einen Gelenkmechanismus mit einer Oberschenkelstruktur verbunden ist. Ein Verriegelungsmechanismus ist vorgesehen, der mittels Fußkraft betätigbar ist und beim Auftreten den Verriegelungsmechanismus aktiviert.

Die 299 14 375 U1 beschreibt ein Orthesengelenk mit einer Oberschenkelschiene und einer Unterschenkelschiene, die einseitig über einen oder mehrere gemeinsame Zapfen oder durch endseitige Verzahnungen im Orthesengelenk miteinander in Eingriff stehen. In dem Orthesengelenk sind Endanschläge vorhanden, die über einen Winkelbereich einstellbar sind. Bei Beugung oder Streckung in dem Orthesengelenk kann eine konstante oder dynamische Bremsung ausgeübt werden, die pneumatisch, hydraulisch oder durch ein elastisches Element erfolgt. Die Bremsung erfolgt insbesondere über elastische Anschläge.

Die DE 600 15 384 T2 beschreibt eine Unterstützungsvorrichtung, die die Existenz oder Funktion eines Gliedes ersetzt und aus mindestens zwei, durch ein künstliches Gelenk miteinander verbundenen Teilen und einer Kontrollvorrichtung für das Gelenk besteht. Ein Sensor ist vorgesehen, der einen Neigungswinkel bezogen auf eine feststehende Linie eines mit dem Gelenk verbundenen Teils erfasst. Der Sensor ist an die Kontrollvorrichtung gekoppelt, die so angeordnet ist, dass sie das Gelenk auf der Basis von vom Sensor übermittelten Neigungswinkeldaten beeinflusst. Neben einer Prothese kann die Unterstützungsvorrichtung auch als eine Orthese ausgebildet sein.

Die DE 39 11 780 A1 beschreibt eine Vorrichtung zur Unterstützung von Bewegungen in Gestalt einer Orthese. Manschetten, die gelenkig miteinander verbunden sind, sind durch mindestens einen Kraftspeicher miteinander gekoppelt. Durch den Kraftspeicher, beispielsweise in Gestalt einer Gasdruckfeder, soll die Bewegung der entsprechenden Körperglieder erleichtert werden.

Die US 6,377,178 B1 beschreibt unter anderem eine Beinorthese mit einem Kniegelenk und einem Knöchelgelenk. Es sind Sensoren vorgesehen, die Belastungen auf das Gewebe während des Tragens der Orthese erfassen.

Die WO 01/43669 A1 betrifft eine Prothese, die mit einem Sensor ausgestattet ist, mit der die Position im Verhältnis zu einer festgesetzten Linie, beispielsweise der Schwerkraftrichtung, detektiert. Auf der Basis der ermittelten Positionsdaten wird über eine Steuerungsvorrichtung das Gelenk beeinflusst. Es sind Befestigungspunkte für eine Aktuatoreinheit vorgesehen, die gemeinsam mit der Gelenkachse der Prothese ein Dreieck ausbilden. Es ist erwähnt, dass eine entsprechende Einrichtung auch als Orthese ausgebildet sein kann.

Die US 5,252,102 A1 beschreibt unter anderem eine Kniegelenksorthese mit einem Oberschenkelteil und einem Unterschenkelteil sowie einem Aktuator, der an dem Oberschenkelteil und dem Unterschenkelteil befestigt ist. Über den Aktuator wird das Unterschenkelteil relativ zu dem Oberschenkelteil verschwenkt, eine Steuerungseinheit überwacht die Position, Geschwindigkeit und Richtung der Bewegung. Die Befestigungspunkte des Aktuators bilden zusammen mit dem Drehpunkt der Orthese ein Dreieck.

Die teilweise sehr komplexen Vorrichtungen benötigen viel Platz, insbesondere in medio-lateraler Ausrichtung. Aufgabe der vorliegenden Erfindung ist es, eine Knieorthese bereitzustellen, mit der neben einer ausreichenden Funktionalität auch eine erhöhte kosmetisch Qualität erreicht werden kann.

Erfindungsgemäß wird diese Aufgabe durch eine Knieorthese mit den Merkmalen des Anspruchs 1 gelöst. Vorteilhafte Ausgestaltungen und Weiterbildungen der Erfindung sind in den jeweiligen Unteransprüchen beschrieben.

Die erfindungsgemäβe Knieorthese mit einer Oberschenkelstruktur, die eine Befestigungseinrichtung zur Festlegung an einem Oberschenkel aufweist, einer Unterschenkelstruktur, die über eine Gelenkeinrichtung mit der Oberschenkelstruktur verschwenkbar gekoppelt ist und eine Befestigungseinrichtung zur Festlegung an einem Unterschenkel sowie ggf. ein Fußteil zur Abstützung eines Fußes aufweist, und einer Aktuatoreinheit zwischen der Oberschenkelstruktur und der Unterschenkelstruktur sieht vor, dass die Befestigungspunkte des Aktuators an den Schienen und der Drehpunkt der Gelenkeinrichtumg ein Dreieck ausbilden und die Befestigungspunkte dergestalt an den Schienen angeordnet sind, dass in einer Kniewinkelstellung im Bereich einer Unterschenkelbeugung zwischen 0° und 90°, insbesondere 10° und 90°, bevorzugt zwischen 30° und 90° zu dem Oberschenkel die Verbindungslinie zwischen den Befestigungspunkten senkrecht auf einer Verbindungslinie zwischen einem Befestigungspunkt und dem Drehpunkt steht Die Abstände zwischen den Befestigungspunkten der Aktuatoreinheit an den Schienen und dem Drehpunkt der Gelenkeinrichtung sind feststehend. Durch eine Relativbewegung der Befestigungspunkt zueinander aufgrund der Verschwenkung der Oberschenkelstruktur relativ zu der Unterschenkelstruktur verändert sich jedoch die Länge der Aktuatoreinheit, beispielsweise eines Hydraulkdämpfers oder eines hydraulischen Antriebs. Durch eine entsprechende Anordnung der Befestigungspunkte an den Schienen ist es möglich, eine kniewinkelabhängige Momentencharakteristik der Knieorthese auch bei einer konstanten Dämpfung der linearen Aktuatoreinheit bereitzustellen. Dies geschieht über eine Anordnung und Ausgestaltung einer Hebellänge in einem bestimmten Winkel zur Normalen der anderen Schiene, also beispielsweise dem Hebel an der Unterschenkelstruktur im Verhältnis zur Normalen der Oberschenkelstruktur, die durch den Drehpunkt der Gelenkeinrichtung hindurch geht. Über den entsprechenden Winkel wird festgelegt, wie sich die effektive Hebellänge für den Aktuator oder Dämpfer, abhängig von dem Kniewinkel, ändert. Dadurch kann der Kniewinkel, bei dem das erzeugbare Widerstandsmoment oder die übertragbaren Kräfte maximal sind, eingestellt werden. Somit ergibt sich ein angepasster Kraftwirkungs- oder Widerstandsmomentenverlauf mit einem nicht linearen Zusammenhang, so dass sich aufgrund der biomechanischen Hebelverhältnisse eine maximale Unterstützung des Beines bei einem gebeugten Bein erreichen lässt, wobei die von dem Aktuator aufgebrachte Kraft konstant gelassen werden kann. Eine maximale Unterstützung des Beines erfolgt somit dann, wenn die Belastung auf die Gelenkeinrichtung bzw. auf das Kniegelenk sehr groß ist, beispielsweise bei einer Winkelstellung des Oberschenkels zum Unterschenkel von 35° bis 45°. Es ist aber auch vorgesehen, bei einer geringeren oder höheren Beugung das maximale Widerstandsmoment oder die maximale Aktuatorkraft bereitzustellen, sofern dies erforderlich oder vom Patienten gewünscht ist. Aufgrund des nicht linearen Verlaufes des Widerstandsmomentes trotz einer konstanten Dämpfung in der Aktuatoreinheit ergibt sich insbesondere im Bereich der Standphasenflexion eine progressive Dämpfung und damit eine progressive Standphasendämpfung, die ohne eine aufwendige Steuerungsmechanik und Sensorik realisiert werden kann, wenn der Aktuator als linear wirkender Dämpfer ausgebildet ist. Entsprechendes gilt für die Ausgestaltung des Aktuators als Antrieb. Von dem Patienten wird ein progressiver Verlauf der Widerstandsmomente in der Standphasenflexion als angenehm beschrieben, da kein spontanes Wegknicken des Kniegelenkes befürchtet werden muss.

Es ist eine Gelenkskinematik vorgesehen, über die die Aktuatoreinheit an der Ober- und/oder Unterschenkelstruktur angelenkt ist. Die Aktuatoreinheit kann auch unmittelbar an der Ober- und/oder Unterschenkelstruktur angelenkt sein, wobei in beiden Fällen vorgesehen ist, dass eine Verschwenkbarkeit und/oder Verlagerbarkeit der Gelenkskinematik bzw. der Aktuatoreinheit gegeben ist. Die Verschwenkbarkeit oder Verlagerbarkeit ist in der Lateralebene gegeben, wobei die Befestigungspunkte der Aktuatoreinheit an der Ober- und Unterschenkelstruktur und der Drehpunkt der Gelenkseinrichtung weiterhin ein Dreieck ausbilden.

Eine Weiterbildung der Erfindung sieht vor, dass der Aktuator ein hydraulischer Dämpfer ist, wobei die Ventile oder Steuereinrichtung des Hydraulikdämpfers in Gehrichtung vor und/oder hinter einer Kolbenzylinderanordnung angeordnet sind. Durch diese Anordnung der Ventile und Steuereinrichtungen sowie der Überströmkanäle ist es möglich, einen relativ schmal bauenden Dämpfer bereitzustellen, so dass die Komponenten in medio-lateraler Ausrichtung wenig auftragen. Dies führt zu einer erhöhten Akzeptanz der Orthese, da diese unter normaler Kleidung getragen werden kann. Die Aktuatoreinheit ist insbesondere als linearer Dämpfer ausgebildet, wodurch sich die gesamte Knieorthese einfacher und preiswerter herstellen lässt.

Die Aktuatoreinheit ist bevorzugt seitlich neben dem Ober- oder Unterschenkel angeordnet, bevorzugt neben dem Oberschenkel, wobei eine Anordnung an der Orthese in Gehrichtung hinter den Schienen bevorzugt wird. Dadurch kann die Hydraulikeinchtung ohne Umlenkeinrichtungen auf Druck belastet werden, wobei durch die Anordnung der Befestigungspunkte des Aktuators an den Schienen leicht eine Anpassung an die gewünschte Momentencharakteristik erfolgen kann. Beispielsweise können mehrere Befestigungspunkte an einer Schiene angeordnet sein, um so die Momentencharakteristik individuell anpassen zu können.

Eine Weiterbildung der Erfindung sieht vor, dass die Aktuatoreinheit an zumindest einem Befestigungspunkt über eine Lagerung mit mindestens zwei Freiheitsgraden gelagert. Speziell ist vorgesehen, dass der Aktuator an zumindest einem Befestigungspunkt über eine kardanische Lagerung mit sich schneidenden oder sich nicht schneidenden Achsen gelagert ist. Aufgrund der individuellen Ausgestaltung der jeweiligen Orthese, die an den Patienten angepasst werden muss, kommt es zu Fluchtungsfehlern bei den Bewegungsachsen, sowohl hinsichtlich der Gelenkachse als auch der Achsen an den Befestigungspunkten, so dass der Zylinder der Aktuatoreinheit sich auf einer Kegelmantelfläche bewegt. Da insbesondere ein Hydraulikdämpfer nur axiale Lasten aufnehmen sollte, um die Haltbarkeit der Aktuatoreinheit zu gewährleisten, ist eine kardanische Lagerung an zumindest einem Befestigungspunkt vorgesehen, idealerweise ist eine Lagerung mit zwei Freiheitsgraden an beiden Lagerungsstellen vorgesehen. Aufgrund der hohen, zu übertragenden hydraulischen Kräfte müsste eine herkömmliche kardanische Lagerung mit sich schneidenden Achsen sehr massiv ausgeführt werden. Aufgrund des limitierten Bauraumes ist es jedoch vorgesehen, die kardanische Lagerung aufzulösen und die Achsen sich nicht schneiden zu lassen. Dies bringt Vorteile für das Design des Gelenkes mit sich, da Kollisionen zwischen der Hydraulik und einem Lagerblock leichter vermieden werden können.

Weiterhin ist es möglich, an dem Verbindungsteil zwischen den Achsen die Hydraulikkraft, die vom Kniemoment abhängt, zu messen, wobei diese Messgröße beispielsweise zur Steuerung des Aktuators eingesetzt werden kann. Mit einer solchen Gelenklagerung ist es möglich, die auftretenden, sehr hohen hydraulischen Kräfte aufzunehmen und gleichzeitig die Baugrößen gering zu halten. Alternativ dazu ist vorgesehen, dass zumindest eine der Lagerstellen als eine Kugelgelenklagerung ausgebildet ist, um einerseits eine geringe Bauhöhe und andererseits ein rein punktuelle, axiale Krafteinleitung in die Aktuatoreinheit zu erreichen. Störende Biegemomente, insbesondere für eine Kolben-Zylinder-Hydraulik störende Momente, können dadurch ausgeschlossen oder verringert werden.

Eine verbesserte Anpassung an die anatomischen Gegebenheiten wird erreicht, wenn der Aktuator in der Lateralebene um einen bestimmten Winkel verkippt angeordnet ist. Dadurch kann der Aktuator oder Hydraulikdämpfer an dem Oberschenkel dicht anliegend montiert werden.

Ergänzend oder auch in Alleinstellung kann das Fußteil eine Dorsalfeder aufweisen, an der oder in der zumindest ein Sensor zur Erfassung der wirksamen Kräfte, z.B. der Biegebelastung und dadurch zur Erfassung des Knöchelmomentes angeordnet ist Diese Sensordaten können genutzt werden, um die Wirkung der Dämpfereinheit über einen Aktuator zu verändern und die Dämpfung zusätzlich zu der konstruktiven Dämpfungscharakteristitk zu verändern. Bei Ausgestaltung der Aktuatoreinheit als Antrieb oder einer Kombination aus Antrieb und Dämpfer werden die Daten genutzt, um das Verhalten der Aktuatoreinheit zu verändern. Ebenfalls können ein Kniewinkelsensor, Kniemomentsensor sowie ein Absolutwinkelsensor zur Erfassung der Orientierung zumindest einer der Schienen im Raum einer Orthese angebracht sein. Die Daten der Sensoren können zusammen oder separat zur Steuerung der Aktuatoreinheit genutzt werden. Eine solche Anordnung von Sensoren zur Steuerung der Widerstandsmomentenverläufe oder des Verhaltens der Aktuatoreinheit kann auch unabhängig von der oben beschriebenen geometrischen Anordnung der Aktuatoreinheit eingesetzt werden.

Die Steuereinrichtung zur Veränderung des Verhaltens der Aktuatoreinheit ist mit den Sensoren gekoppelt und dient zur Veränderung z.B. des Dämpferwiderstandes oder der aufgebrachten Kraft in der Aktuatoreinheit.

Die Bewegungsachse der Orthese zwischen der Ober- und Unterschenkelstruktur kann durch die Mittelpunkte zweier Kugelgelenke festgelegt werden, wenn die Ober- und Unterschenkelstruktur über Kugelgelenke miteinander verbunden sind Alternativ können die Ober- und Unterschenkelstruktur auch über ein Kardangelenk miteinander verbunden sein.

Der Schwenkwinkel der Gelenksmechanik in der Lateralebene kann durch ein Mitläufergelenk festgelegt werden, bei dem es sich um eine Schamier-, Kardan- oder Kugelgelenk handeln kann.

Der Hebel zwischen der Drehachse der Gelenkeinrichtung und dem dazu benachbarten Befestigungspunkt der Aktuatoreinheit kann durch zwei über mindestens einen Lenker gekoppelte Kugelgelenke realisiert werden.

Das Verfahren zur Steuerung einer Knieorthese mit einer Oberschenkelstruktur, einer Gelenkeinrichtung und einer Unterschenkelstruktur, die ein Fußteil aufweist, sieht vor, dass ein wirksames Moment, insbesondere Knöchelmoment, innerhalb der Orthese bestimmt und der Widerstand der Aktuatoreinheit in Abhängigkeit von dem Moment, insbesondere Knöchelmoment verändert wird. Ergänzend oder alternativ kann die Aktuatoreinheit in Abhängigkeit von einem gemessenen oder errechneten Kniemoment, dem Kniewinkel, also der Stellung von Oberschenkelstruktur und Unterschenkelstruktur zueinander, oder von der Raumorientierung zumindest einer Oberschenkel- oder Unterschenkelstruktur verändert werden. Dadurch ist es möglich, eine Kontrolle der Stand- und Schwungphase bei Patienten mit Lähmungen, bei denen das Bein nicht mehr willentlich kontrollierbar ist, zur Verfügung zu stellen. Durch das veränderliche Widerstandsmoment, beispielsweise über einen hydraulischen Aktuator, kann jedes beliebige Kniemoment generiert werden, wodurch Standphasenflexion und alternierendes Bergab- und Treppabgehen ermöglicht werden. Bei aktiven Aktuatoreinheiten kann eine entsprechende Unterstützung der Bewegung gezielt und effizient erfolgen.

Eine Weiterbildung des Verfahrens sieht vor, dass die innerhalb der Orthese generierten Sensorsignale zusätzlich zur Erzeugung von Impulsen zur funktionellen Elektrostinlulation der verbliebenen Restmuskulatur verwendet werden. Hierbei werden die von der Orthese ermittelten Messwerte zur Festlegung der Stärke bzw. des Timings der Stimulationsimpulse einer mit dem Steuergerät verbundenen Einheit für funktionelle Elektrostimulation verwendet. Damit können die verbliebenen Muskeln gezielt aktiviert werden, um den Bewegungsablauf zu verbessern und die Aktuatoreinrichtung zu unterstützen oder zu ersetzen.

Nachfolgend wird ein Ausführungsbeispiel der Erfindung anhand der beigefügten Figuren näher erläutert. Es zeigen:
- Figur 1 -: eine schematische Seitenansicht einer Knie-Knöchel-Fuß-Orthese in Seitenansicht;
- Figur 2 -: eine schematische Darstellung der Orthese in Frontalansicht;
- Figur 3 -: eine Ortheseneinrichtung in Dorsalansicht;
- Figuren 4a und 4b -: schematische Orthesendarstellungen stehend und sitzend;
- Figur 5 -: eine Prinzipskizze der Aktuatoreinheitanordnung;
- Figur 6 -: ein Momentenverlauf über die Kniebeugung;
- Figur 7 -: Positionen der Aktuatoreinheit bei verschiedenen Beugewinkeln;
- Figur 8 -: eine Variante der Figur 1;
- Figur 9 -: einen Sensor auf einer Trägerplatte;
- Figur 10 -: ein Anordnungsbeispiel von Sensoren gemäß Figur 9 in einem Verbindungselement;
- Figur 11 -: eine Variante des Sensors gemäß Figur 9; sowie
- Figur 12 -: ein Anordnungsbeispiel des Sensors gemäß Figur 11.

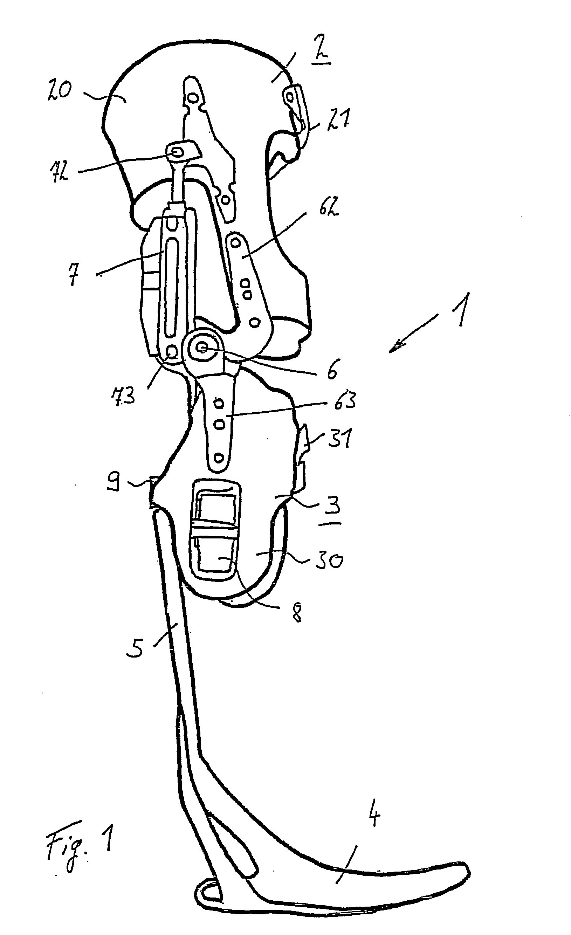

In der Figur 1 ist in einer schematischen Darstellung eine Ortheseneinrichtung 1 in Gestalt einer Knie-Knöchel-Fuß-Orthese dargestellt. Die Ortheseneinrichtung 1 weist ein Oberteil 2 in Gestalt einer Oberschenkelstruktur sowie ein Unterteil 3 in Gestalt einer Unterschenkelstruktur auf. Die Oberschenkelstruktur 2 und die Unterschenkelstruktur 3 sind über eine Gelenkeinrichtung 6 um eine Gelenkachse schwenkbar miteinander verbunden. An der Unterschenkelstruktur 3 ist ein Fußteil 4 über ein Verbindungselement 5 befestigt. Das Verbindungselement 5 ist in einer Aufnahmeeinrichtung 9 dorsal an der Unterschenkelstruktur 3 angeordnet.

Die Oberschenkelstruktur 2 besteht aus einem Grundkörper in Gestalt einer Aufnahmeschale, die über Befestigungsmittel 21 an einem Oberschenkel festgelegt werden kann. Die Befestigungsmittel sind beispielsweise Schnallen, Laschen oder Klettverschlüsse, die über eine Öffnung innerhalb der Oberschenkelstruktur 2 gelegt werden können und so die Oberschenkelstruktur 2 verschließen. Die Öffnung kann frontal oder dorsal sein. Ebenfalls sind an der Unterschenkelstruktur Befestigungsmittel 31 angeordnet, die sich frontal unterhalb des Kniegelenkes um den Unterschenkel schließen lassen.

Die Gelenkeinrichtungen 6 können sowohl medial als auch lateral an der Ortheseneinrichtung 1 angeordnet sein, wobei die Oberschenkelstruktur 2 und die Unterschenkelstruktur 3 über Gelenkstrukturelemente 62, 63 an der Gelenkeinrichtung 6 befestigt sind. An der Unterschenkelstruktur 3 ist ein Steuergerät 8 zur Steuerung einer Aktuatoreinheit 7 angeordnet, die auf Grundlage eines Programmes und verschiedener Sensordaten Parameter innerhalb der Aktuatoreinheit verändert, um so die Dämpfung zu erhöhen oder zu verringern oder die aufgebrachte Kraft anzupassen. Alternativ zu der Anbringung der Steuereinheit 8 an dem Unterschenkelstruktur 3 kann diese auch an der Oberschenkelstruktur 2 angeordnet sein. Die Aktuatoreinheit 7 ist als eine hydraulische oder pneumatische Aktuatoreinheit mit einem Kolben und einem Zylinder ausgebildet. Im dargestellten Ausführungsbeispiel ist das proximale Ende einer Kolbenstange an einem oberen Anlenkpunkt 72 an der Oberschenkelstruktur 2 und ein unterer Anlenkpunkt 73 eines Aktuatorgehäuses als distaler Lagerungspunkt der Aktuatoreinheit 7 an einem proximalen Ende der Unterschenkelstruktur 3 angeordnet, im Bereich der unteren Gelenkstrukturelemente 63. Das Fußteil 4 kann einstückig mit dem Verbindungselement 5 ausgebildet und auswechselbar an dem Unterteil 3 in einer Aufnahmeeinrichtung 9 befestigt sein, alternativ kann das Verbindungselement 5 auch an einem separat ausgebildeten Fußteil 4 befestigt sein. Das Verbindungselement 5 kann auch einstückig mit der Unterschenkelstruktur 3 ausgebildet sein.

In dem dargestellten Ausführungsbeispiel ist die Aktuatoreinheit 7 nur an der lateralen Seite der Ortheseneinrichtung 1 angeordnet, als Alternative zu einer lateralen und medialen Anordnung zweier Gelenkeinrichtungen 6 mit ggf. zwei Aktuatoreinheiten 7.

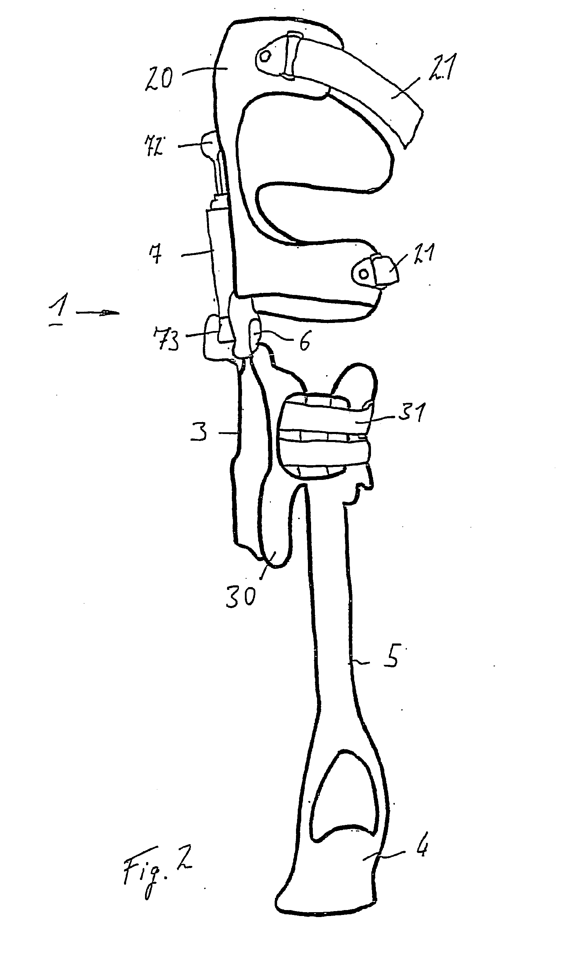

In der Figur 2 ist die Ortheseneinrichtung 1 gemäß Figur 1 in Frontalansicht dargestellt. Hier ist zu erkennen, dass die Oberschenkelstruktur 2 aus einem schalenförmigen Grundkörper 20 besteht, an dem Befestigungseinrichtungen 21 in Gestalt von Gurten angeordnet sind. Entsprechend ist die Unterschenkelstruktur 3 mit einem Grundkörper 30 ausgestattet, der über die Befestigungseinrichtung 31 an dem nicht dargestellten Unterschenkel eines Orthesenträgers festgelegt werden kann. In der Figur 2 ist eine Ortheseneinrichtung 1 für ein rechtes Bein dargestellt, die Gelenkeinrichtung 6 ist lateral und einseitig an der Ortheseneinrichtung 1 angeordnet. Der Figur 2 kann ebenfalls entnommen werden, dass die Grundkörper 20, 30 der Oberschenkelstruktur 2 bzw. Unterschenkelstruktur 3 dorsal geschlossen ausgebildet sind.

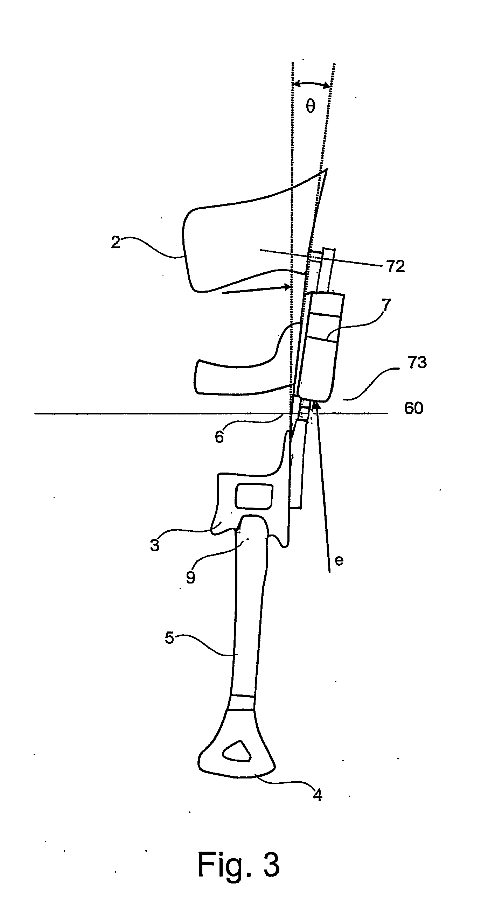

In der Figur 3 ist die Ortheseneinrichtung 1 in rückseitiger Ansicht dargestellt. Durch die Gelenkeinrichtung 6 verläuft eine mit einer anatomischen Kompromissdrehachse fluchtende Gelenkachse 60, um die das Oberteil 2 und das Unterteil 3 zueinander verschwenkbar gelagert sind. Die Gelenkachse 60 ist in der Figur 3 waagerecht dargestellt, was in der Realität nur ausnahmsweise der Fall wäre. Unmittelbar an der Oberschenkelstruktur 2 ist die Aktuatoreinheit 7 angeordnet. Anhand der Figur 3 ist zu erkennen, dass die Längserstreckung der Aktuatoreinheit 7 in einem Winkel θ zu der Vertikalen verläuft, so dass die Achsen der proximalen und distalen Aktuatoraufhängungen 72, 73 nicht parallel zu der Knieachse verlaufen. Dieser Winkel θ entspricht dem Ilio-Tibia-Winkel, um den die Aktuatoreinheit 7 aus der Sagittalebene herausgeschwenkt werden muss. Die Schwenkachse verläuft dabei in anterior-posterior-Richtung. Der Ilio-Tibia-Winkel θ kann bis zu 30° betragen, je nach Art der Befestigung und den anatomischen Gegebenheiten des Orthesenträgers.

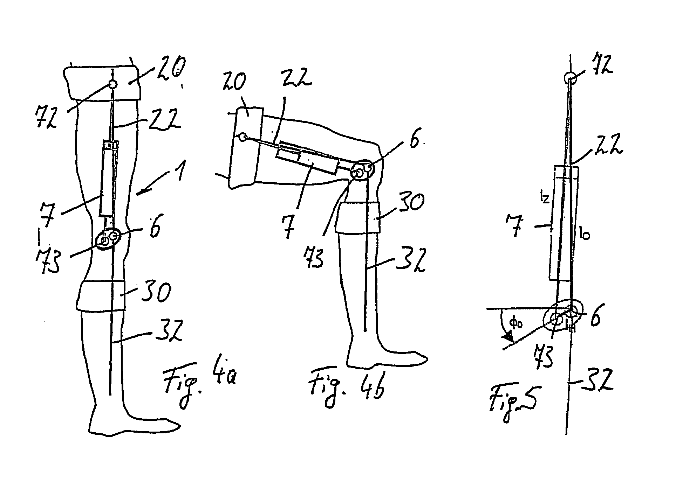

Die Figuren 4a und 4b zeigen eine schematische Darstellung einer angelegten Ortheseneinrichtung 1, einmal in stehender Position in Figur 4a und einmal in sitzender Position in Figur 4b. Die Ortheseneinrichtung 1 ist vereinfacht mit einer Oberschenkelschiene 22 und eine Unterschenkelschiene 32 dargestellt, die um die Gelenkeinrichtung 6 schwenkbar zueinander gelagert sind. Die Oberschenkelschiene 22 ist über eine entsprechende Befestigungseinrichtung 20 an dem Oberschenkel festgelegt, während die Unterschenkelschiene 32 über die entsprechende Befestigungseinrichtung 30 an dem Unterschenkel festgelegt ist. Die Oberschenkelschiene entspricht der Oberschenkelstruktur, während die Unterschenkelschiene der Unterschenkelstruktur entspricht. Die Aktuatoreinheit 7 ist lateral und dorsal zu den Schienen 22, 32 angeordnet, wobei der obere Befestigungspunkt 72 der Aktuatoreinheit 7 an der Oberschenkelschiene 22 und der untere Befestigungspunkt 73 der Aktuatoreinheit 7 an der Unterschenkelschiene 32 angeordnet ist. Die Gelenkeinrichtung 6 bildet gleichzeitig den Drehpunkt der beiden Schienen 22, 32 zueinander aus. Der untere Befestigungspunkt 73, der die distale Lagerstelle der Aktuatoreinheit 7 bildet, ist dorsal zu dem Drehpunkt der Gelenkeinrichtung 6 beabstandet, wobei dieser Abstand über einen schräg nach unten gerichteten Hebel realisiert wird. In der dargestellten Ausführungsform liegt der Befestigungspunkt 73 der Aktuatoreinheit 7 bei senkrechter Unterschenkelschiene 32 unterhalb des Drehpunktes der Gelenkeinrichtung 6.

In der Figur 4b ist schematisch der sitzende Zustand eines Orthesenträgers mit angelegter Orthese gezeigt. Der Winkel zwischen der Oberschenkelschiene 22 und der Unterschenkschiene 32 ist größer als 90°. Der Zeichnung 4b ist zu entnehmen, dass die Aktuatoreinheit 7 seitlich neben dem Oberschenkel des Orthesenträgers angeordnet ist. Dadurch ist es möglich, dass das Bein vollständig gebeugt werden kann, ohne dass die Aktuatoreinheit 7 ein Hindernis darstellen würde. Ebenfalls trägt bei einer solchen Anordnung der Aktuatoreinheit 7 die Orthese wenig auf, so dass ein möglichst unauffälliges Tragen der Orthese möglich ist. In den Figuren 4a und 4b ist das Fußteil zur Abstützung des Fußes nicht dargestellt. Grundsätzlich besteht auch die Möglichkeit, die Orthese 1 ohne ein Fußteil auszustatten.

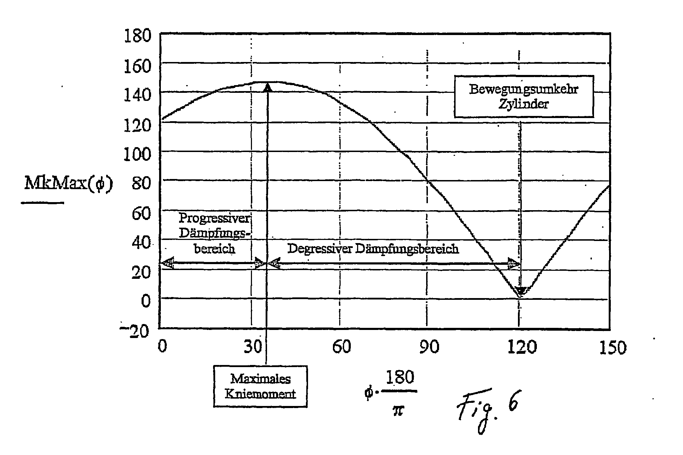

Die Figur 5 zeigt die prinzipielle geometrische Anordnung der Aktuatoreinheit 7 an den Schienen 22, 32. Der Hydraulikkolben ist an seinem oberen Ende an dem oberen Lagerungspunkt 72 gelagert, beispielsweise in einem Kugelgelenklager. An dem unteren Ende der Aktuatoreinheit 7 erfolgt die Abstützung an der unteren Lagerstelle 73, die mit der unteren Unterschenkelschiene 32 gekoppelt ist. Die Oberschenkelschiene 22 und die Unterschenkelschiene 32 bzw. das Oberteil und das Unterteil der Ortheseneinrichtung sind in der Gelenkeinrichtung 6 schwenkbar um eine Gelenkachse miteinander gekoppelt. Der Befestigungspunkt 73 der unteren Lagerungsstelle ist um die Strecke l_{H} dorsal zu dem Drehpunkt bzw. der Drehachse der Gelenkeinrichtung 6 versetzt angeordnet, so dass die beiden Befestigungspunkte 72, 73 und der Drehpunkt der Gelenkeinrichtung 6 ein Dreieck bilden. Der Schenkel zwischen dem unteren Befestigungspunkt 73 und dem Drehpunkt 6 steht in einem Winkel Φ₀ von ca. 30° zu dem Schenkel, der zwischen dem oberen Befestigungspunkt 72 und dem Drehpunkt 6 ausgebildet ist, wenn die Ortheseneinrichtung 1 gestreckt ist. Erst nach Verschwenken der Oberschenkelstruktur 2 bzw. der Oberschenkelschiene 22 um einen Winkel, der Φ₀ in etwa entspricht, in Beugerichtung steht die Aktuatoreinheit 7 bzw. der Schenkel zwischen dem oberen Befestigungspunkt 72 und dem unteren Befestigungspunkt 73 senkrecht auf der Verbindungslinie l_{H} zwischen dem unteren Befestigungspunkt 73 und dem Drehpunkt der Gelenkeinrichtung 6. Der Hebel l_{H} zwischen der Drehachse der Gelenkeinrichtung 6 und dem dazu benachbarten Befestigungspunkt 73 der Aktuatoreinheit 7 kann durch zwei über mindestens einen Lenker gekoppelte Kugelgelenke realisiert werden. Dieser Winkel Φ₀ liegt zwischen 0° und 90°, insbesondere zwischen 10° und 90° und bevorzugt zwischen 30° und 90° und wird so gewählt, dass er dem Winkel entspricht, in dem das maximale Kniemoment zwischen dem Oberschenkel und dem Unterschenkel des Orthesenträgers erreicht wird oder werden soll. Ein schematischer Verlauf des Kniemomentes über den Beugewinkel ist in der Figur 6 dargestellt. Als Winkel Φ₀ ist ein Winkel von 30° angenommen. Der Figur 6 ist zu entnehmen, dass zwischen der gestreckten Stellung bei 0° bis zur Beugung bis zu einem Winkel von 35° ein progressiver Dämpfungsbereich vorliegt, so dass auch bei einer linearen Ausgestaltung der Aktuatoreinheit 7 eine progressive Dämpfung aufgrund der sich verändernden Geometrie erzielt wird. Die Abweichung des Endwinkels des progressiven Dämpfungsbereiches von Φ₀ ergibt sich aus der Veränderung der Längen der Aktuatoreinheit während der Beugung.

Zu Beginn der Beugung, also über die ersten 35° Beugewinkel, steigt das Kniemoment bis zu einem Winkel Φ₀, der sich aufgrund der trigonometrischen Beziehungen ergibt und in etwa Φ₀ entspricht, an, bis die Verbindungslinie zwischen dem oberen Gelenkpunkt 72 und dem unteren Gelenkpunkt 73 senkrecht auf der Verbindungslinie zwischen dem unteren Gelenkpunkt 73 und dem Drehpunkt der Gelenkeinrichtung liegt. Aufgrund der reinen Axialkraft, die die Aktuatoreinheit 7 einer Kniebeugung entgegen setzen kann, wird ein maximales Kniemoment dann erreicht, wenn die Wirksachse der Aktuatoreinheit 7 senkrecht auf der Verbindungslinie zwischen dem Drehpunkt der Gelenkeinrichtung 6 und dem unteren Lagerpunkt 73 steht. Bei einer weiteren Beugung des Knies verringert sich das Kniemoment um den Sinus der weiteren Verschenkung, bis es zu einer Bewegungsumkehr der Aktuatoreinheit kommt, also wenn die Beugung größer als 90° plus Φ₀ ist. Dann steigt das Kniemoment mit dem Kosinus weiter an, sofern die Wirkung des Zylinders der Aktuatoreinheit oder des Dämpfers von Druck auf Zug umgeschaltet wurde.

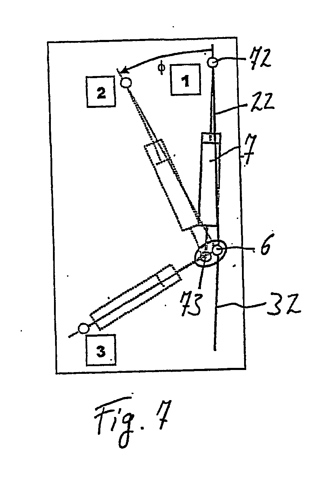

In der Figur 7 sind drei extreme Zustände gezeigt. Zustand 1 liegt bei einer gestreckten Stellung des Beines vor. In Stellung 2 fand eine Verschwenkung um den Winkel Φ statt, so dass das maximale Kniemoment erreicht ist. In der Stellung 3 liegt die Wirksachse der Aktuatoreinheit 7 auf der Verbindungslinie zwischen dem unteren Lagerpunkt 7 und dem Drehpunkt der Gelenkeinrichtung 6, so dass ein Verschwenkwinkel von 90° plus Φ erreicht ist. In dieser Stellung ist das wirksame Kniemoment gleich 0, da kein Hebel vorhanden ist und die Aktuatorkraft senkrecht durch den Drehpunkt der Gelenkeinrichtung 6 verläuft.

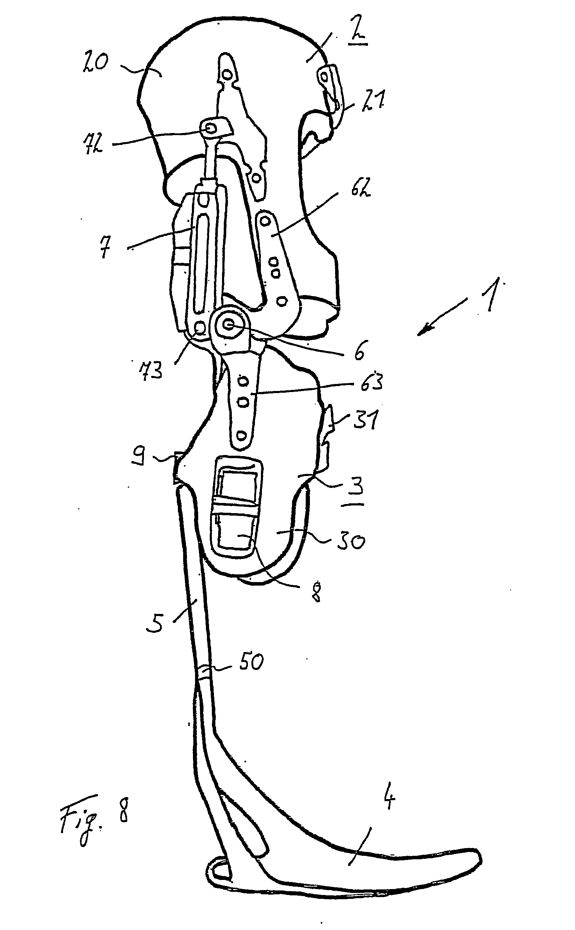

Neben der Beeinflussung des Momentenverlaufes aufgrund der konstruktiven Ausgestaltung und Anordnung der Aktuatoreinheit 7 innerhalb der Gelenkorthese 1 und die spezielle Anordnung der Lagerungspunkte 72, 73 relativ zu dem Drehpunkt der Gelenkeinrichtung 6 bzw. zu der Gelenkachse 60, kann die Dämpfung innerhalb der Gelenkorthese auf der Grundlage von Sensordaten kontrolliert werden. Der Sensor ist dabei bevorzugt in dem Verbindungselement 5, das als Dorsalfeder ausgebildet ist, angeordnet und ermittelt das während des Stehens oder Gehens wirksame Knöchelmoment. Die Anordnung des Sensors 50 ist dabei in der Figur 8 dargestellt, die im Wesentlichen der Figur 1 entspricht, mit dem Unterschied, dass der Sensor 50 an oder in der Dorsalfeder 5, die als Verbindungselement zwischen dem Fußteil 4 und der Unterschenkelstruktur 3 ausgebildet ist, angeordnet ist. Der Sensor 50 übermittelt die Daten an die Steuerungseinheit 8, über die dann wiederum Ventile innerhalb der Aktuatoreinheit 7 angesteuert werden. Ebenfalls können Drosselstellen vergrößert oder verkleinert werden, so dass unterschiedliche Fluidströme und damit Dämpfungsgrade oder Kräfte während der Bewegung realisiert werden können. Eine Dämpfung kann auch über elektrorheologische oder magnetorheologischen Fluide und eine entsprechende Veränderung der Viskosität dieser Fluide erreicht werden.

Eine solche Steuerung der Ortheseneinrichtung 10 ermöglicht es insbesondere für Patienten mit Lähmungen, bei denen das Bein vorhanden, jedoch nicht mehr willentlich kontrollierbar ist, dass die Kontrolle der Stand- und Schwungphase während des Gehens erfolgen kann. Durch das insbesondere hydraulische Aktuatorprinzip ist es möglich, dass jedes beliebige Kniedämpfungs- oder -beugemoment generiert werden kann, wodurch eine Standphasenflexion und ein alternierendes Bergab- oder Treppabgehen und eine aktive Unterstützung der Bewegung ermöglicht werden. Dabei ist für die Akzeptanz einer solchen Ortheseneinrichtung wichtig, dass neben der Funktionalität auch ein optisch möglichst unauffälliges Design erreicht wird, insbesondere muss eine Ausdehnung der Ortheseneinrichtung 1 in medio-lateraler-Richtung möglichst minimiert werden.

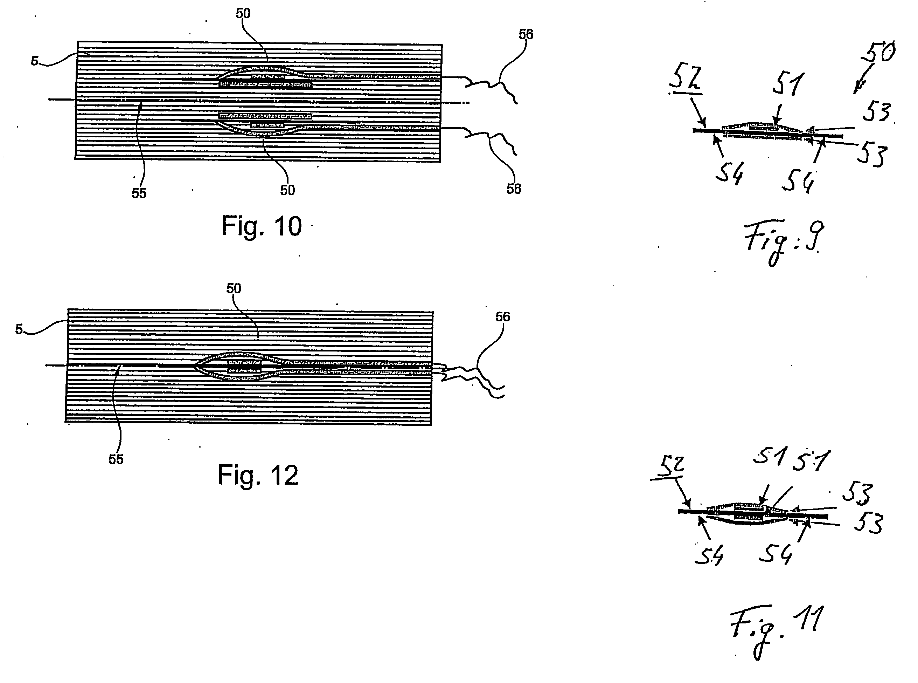

Der Sensor 50 zur Ermittlung der wirksamen Kräfte in dem Verbindungselement 5, insbesondere zur Ermittlung des Knöchelmomentes, kann in das Verbindungselement 5 eingebaut sein. In der Figur 9 ist in einer Querschnittsansicht schematisch die Ausgestaltung eines Sensors 50 mit einem Dehnmessstreifen 51 gezeigt, der auf einem Träger, insbesondere Metallträger 52 befestigt ist. Der Dehnmessstreifen 51 ist in herkömmlicher Weise auf dem Träger 52 befestigt, beispielsweise verklebt, und gegenüber der Umgebung durch Trennschichten 52 abgeschirmt. Die Trennschichten 53 sind an dem Träger 52 festgelegt und können beispielsweise aus einer Trennfolie oder einer Silikoneinbettung oder -beschichtung bestehen. Diese Trennschichten 53, die sowohl auf der Oberseite als auch der Unterseite des Trägers 52 angeordnet sind, verhindern, dass Scherkräfte auf den Träger 52 und den darauf befestigten Dehnmessstreifen 51 übertragen werden. Seitlich neben den Trennschichten 53 sind Verbindungsbereiche 54 des Trägers 52 vorgesehen, an denen der Träger 52 mit dem umgebenen Verbundmaterial verbunden werden kann. Diese Verbindungsbereiche 54 treten in Verbindung mit den Bindemitteln des Faserverbundwerkstoffes und stellen so eine Verbindung zu den Faserwerkstoffen her.

In der Figur 10 ist in einer schematischen Seitenansicht das Verbindungselement 5 beispielsweise in Gestalt eines Balkenfederelementes dargestellt. Beidseitig der neutralen Faser 55 ist je ein Sensor 50 mit einem abgeschirmten Dehnmessstreifen 51 auf einem Träger 52 einlaminiert. Kabel 56, die mit dem Dehnmessstreifen 51 verbunden sind, führen aus dem Verbindungselement 5 heraus und zu einer Auswerteeinheit bzw. der Steuereinrichtung 8. Der Träger 52 ist bevorzugt dünn ausgebildet, beispielsweise zwischen 0,1 mm und 0,5 mm, sodass die gesamte Anordnung des Sensors 50 mit den Dehnmessstreifen 51 und Trennschichten 53 eine Dicke von unter 1 mm aufweist. Die Empfindlichkeit der Sensoren kann durch die Lage innerhalb des Verbindungselementes 5 eingestellt werden, wobei die Veränderung sich durch die Variation der Entfernung zu der neutralen Faser 55 ergibt.

Eine Variante der Erfindung ist in den Figuren 11 und 12 dargestellt, bei der beidseitig auf dem Träger 52 Dehnmessstreifen 51 angeordnet und von Trennschichten 53 umgeben sind. Auch hier sind neben den Trennschichten 53 Verbindungsbereiche 54 vorgesehen, um in das Verbindungselement 5 einlaminiert zu werden. In der Figur 12 ist in Schnittdarstellung die Anordnung des Sensors 50 gemäß Figur 11 innerhalb des Verbindungselementes 5 dargestellt. Die Anordnung des Sensors 50 erfolgt in der neutralen Faser 55 des Verbindungselementes 5, die Kabel 56 führen aus dem Verbindungselement 5 heraus. Der Träger 52 biegt sich zusammen mit dem Verbindungselement 5 und erzeugt ein Ausgangssignal. Aufgrund der unmittelbar zu der neutralen Faser 55 benachbarten Anordnung der Dehnmessstreifen 51 wird ein hoher Verstärkungsfaktor benötigt, jedoch ergibt sich der Vorteil, dass nur ein einziger Fremdkörper innerhalb des Verbundwerkstoffelementes 5 angeordnet werden muss. Dieser Fremdkörper in Gestalt des Sensors 50 kann als vollständig vorgefertigtes Modul, das ggf, eine elektronische Schaltung zur Signalverstärkung und Signalaufbereitung beinhaltet, einfach eingelegt und einlaminiert werden.

Das Verbindungselement 5 ist als eine Dorsalfeder ausgebildet, um die Energieeffizienz für den Orthesenträger zu erhöhen. Die Dorsalfeder 5 kann ebenso wie das daran angebrachte oder angeformte Fußteil 4 aus einer faserverstärkten, elastischen Struktur bestehen, beispielsweise aus einer Karbonstruktur, die die Energie des Kontaktstoßes beim Fersenkontakt aufnehmen und bei der Schwungphaseneinleitung die aufgenommene Energie wieder abgeben, wodurch die Einleitung der Schwungphase erleichtert wird. Durch die Messung der Verformungen innerhalb der Dorsalfeder 5, die durch den integrierten Sensor 50 bzw. mehrere integrierte Sensoren, die auch in dem Fußteil 4 integriert sein können, analog zu der Integration innerhalb der Dorsalfeder 5, ist es möglich, aus der direkten Messung der Verformungen innerhalb der Strukturen auf die Körperposition und die Bewegungsabsicht des Patienten Rückschlüsse zu ziehen. Aufgrund der ermittelten Sensordaten, beispielsweise des Knöchelmomentes oder der innerhalb der jeweiligen Struktur wirksamen Belastungshöhen und Belastungsrichtungen ist es möglich, verschiedene Einstellungen in der Aktuatoreinheit 7 vorzunehmen. Dabei werden verschiedene Zustände der Dämpfung oder des Antriebs geschaltet, wobei die Zustandsübergänge in Abhängigkeit von den Sensorsignalen innerhalb der Dorsalfeder 5 und, soweit notwendig, von einem Kniewinkelsensor durchgeführt werden. Der Kniewinkel, also der Beugewinkel zwischen der Oberschenkelstruktur 2 und der Unterschenkelstruktur 3 kann entweder über einen unmittelbar an der Gelenkeinrichtung 6 angeordneten Winkelmesser oder unter Zuhilfenahme von Sensoren, die die Position der jeweiligen Komponenten relativ zu einer orthesenunabhängigen Richtungskomponente messen, beispielsweise relativ zur Schwerkraftkomponente oder zur Waagerechten. Ebenfalls können Sensoren zur Messung von Kniemomenten oder Axialkraftbelastungen vorgesehen sein, um die Steuerungsmöglichkeiten der Aktuatoreinheit weiter zu verbessern.

Während der Abrollbewegung des Fußes beim Gehen durchläuft das Knöchelmoment während der Standphase einen typischen zeitlichen Verlauf. Im Zuge des Abrollverlaufes wird das Federelement 5 zunächst verformt, also mit Energie "aufgeladen", und anschließend entspannt sich die Feder 5 wieder, also wird Energie abgegeben. Analog dazu ergibt sich für den Kniewinkel und für das Kniemoment ein typischer Verlauf, der über die Aktuatoreinheit 7, insbesondere über die Aktuatorhydraulik, beeinflusst werden kann, wodurch auch der zeitliche Verlauf der in der Feder 5 gespeicherten Energie innerhalb bestimmter Grenzen beeinflusst werden kann.

Die Aktuatoreinheit 7 ist bevorzugt als eine lineare, hydraulische Dämpfereinheit ausgestattet. Die Dämpfung kann über zwei Ventile getrennt für die Extension und Flexion verstellt werden, wobei die Ventile über Servomotoren und ein gegebenenfalls zwischengeschaltetes Getriebe verstellt werden. Die Aufnahmeschalen 20, 30 der Oberschenkel- und Unterschenkelstrukturen 2, 3 bestehen bevorzugt ebenfalls aus einem Kohlefasermaterial, um hohe Kräfte aufnehmen zu können, ohne großen Bauraum zu benötigen und die Ortheseneinrichtung 1 zu schwer zu machen. Die Aktuatoreinheit 7 erzeugt bremsende Momente, indem Bewegungsenergie in Wärme umgewandelt wird. Mit der Ortheseneinrichtung 1 ist es möglich, mit unterschiedlichen Geschwindigkeiten in der Ebene zu gehen, Rampen oder Treppen mit unterschiedlichen Steigungen sowie unterschiedliche Sitzpositionen einzunehmen. Unterschiedliche Betriebsmodi können über entsprechende Bedienelemente an der Ortheseneinrichtung 1 verstellt werden, beispielsweise über eine Fernbedienung oder eine PC-Schnittstelle, über die innerhalb der Steuerungseinheit 8 unterschiedliche Programme aufgerufen werden. Auf eine ähnliche Art und Weise kann auch benötigte Software auf die Steuereinheit 8 aufgespielt oder von einem Orthopädietechniker verändert werden.

## Patentansprüche

1. Knieorthese mit einer Oberschenkelstruktur (2), insbesondere Oberschenkelschiene, die eine Befestigungseinrichtung (21) zur Festlegung an einem Oberschenkel aufweist, einer Unterschenkelstruktur (3), insbesondere Unterschenkelschiene, die über eine Gelenkeinrichtung (6) mit der Oberschenkelstruktur (2) verschwenkbar gekoppelt ist und eine Befestigungseinrichtung (31) zur Festlegung an einem Unterschenkel aufweist, und einer Aktuatoreinheit (7) zwischen der Oberschenkel- und der Unterschenkelstruktur (2, 3), wobei die Befestigungspunkte (72, 73) der Aktuatoreinheit (7) an den Ober- und Unterschenkelstrukturen (2, 3) und der Drehpunkt der Gelenkeinrichtung (6) ein Dreieck ausbilden und die Befestigungspunkte (72, 73) dergestalt an den Strukturen (2, 3) angeordnet sind, dass in einer Kniewinkelstellung im Bereich einer Unterschenkelbeugung zwischen 0° und 90° zu dem Oberschenkel die Verbindungslinie zwischen den Befestigungspunkten senkrecht auf einer Verbindungslinie (IH) zwischen einem Befestigungspunkt (73) und dem Drehpunkt (6) steht, **dadurch gekennzeichnet, dass** die Aktuatoreinheit (7) und/oder die Befestigungspunkte (72, 73) der Aktuatoreinheit (7) in der Lateralebene verschiebbar oder verschwenkbar gelagert sind.

2. Knieorthese nach Anspruch 1, **dadurch gekennzeichnet, dass** Ventile oder Steuereinrichtungen der Aktuatoreinheit (7) in Gehrichtung vor und/oder hinter einer Kolben-Zylinder-Anordnung angeordnet sind.

3. Knieorthese nach Anspruch 1 oder 2, **dadurch gekennzeichnet, dass** die Aktuatoreinheit (7) seitlich neben dem Ober- oder Unterschenkel und/oder in Gehrichtung hinter den Strukturen (2, 3) angeordnet ist.

4. Knieorthese nach einem der voranstehenden Ansprüche, **dadurch gekennzeichnet, dass** die Aktuatoreinheit (7) an zumindest einem Befestigungspunkt (73) über eine Lagerung mit mindestens zwei Freiheitsgraden gelagert ist.

5. Knieorthese nach Anspruch 4 **dadurch gekennzeichnet, dass** die Aktuatoreinheit (7) an zumindest einem Befestigungspunkt über eine kardanische Lagerung mit sich schneidenden, sich nicht schneidenden Achsen oder einer Kugelgelenklagerung gelagert ist.

6. Knieorthese nach einem der voranstehenden Ansprüche, **dadurch gekennzeichnet, dass** die Aktuatoreinheit (7) in der Lateralebene um einen Winkel θ verkippt angeordnet ist.

7. Knieorthese nach einem der voranstehenden Ansprüche, **dadurch gekennzeichnet, dass** das Fußteil (4) eine Dorsalfeder (5) aufweist, an der oder in der zumindest ein Sensor (50) zur Erfassung der wirksamen Kräfte in der Feder (5), insbesondere des Knöchelmomentes angeordnet ist.

8. Knieorthese nach einem der voranstehenden Ansprüche, **dadurch gekennzeichnet, dass** ein Kniemomentsensor und/oder ein Kniewinkelsensor an der Orthese angeordnet sind.

9. Knieorthese nach einem der voranstehenden Ansprüche, **dadurch gekennzeichnet, dass** ein Absolutwinkelsensor zu Erfassung der Orientierung zumindest einer der Strukturen (2, 3) im Raum an der Orthese angebracht ist.

10. Knieorthese nach einem der voranstehenden Ansprüche, **dadurch gekennzeichnet, dass** eine Steuereinrichtung (8) zur Veränderung des Verhaltens der Aktuatoreinheit (7), insbesondere des Dämpfungswiderstandes in Abhängigkeit von Sensorsignalen vorgesehen ist.

11. Knieorthese nach einem der voranstehenden Ansprüche, **dadurch gekennzeichnet, dass** die Bewegungsachse der Gelenkeinrichtung (6) durch Mittelpunkte zweier Kugelgelenke festgelegt ist.

12. Knieorthese nach einem der voranstehenden Ansprüche, **dadurch gekennzeichnet, dass** die Oberschenkelstruktur (2) und die Unterschenkelstruktur (3) über ein Kardan- oder Kugelgelenk miteinander verbunden sind.

## Claims

1. A knee orthosis with a thigh structure (2), in particular a thigh rail, which has a fastening means (21 for securing to a thigh with a lower leg structure (3), in particular a lower leg rail, which is coupled pivotably to the thigh structure (2) via a joint mechanism (6) and has a fastening means (31) for securing to a lower leg, and with an actuator unit (7) between the thigh structure and the lower leg structure (2, 3), where the fastening points (72, 73) of the actuator unit (7) on the thigh structure and the lower leg structure (2, 3), and the center of rotation of the joint mechanism (6) form a triangle, and the fastening points (72, 73) are arranged on the structures (2, 3) in such a way that the connecting line between the fastening points is perpendicular to a connecting line (I_{H}) between a fastening point (73) and the center of rotation (6) in an angular position of the knee in which the lower leg is bent at an angle of between 0° and 90° relative to the thigh, **characterized in that** the actuator unit (7) and/or the fastening points (72, 73) of the actuator unit (7) are mounted so as to be movable or pivotable in the lateral plane.

2. The knee orthosis as claimed in claim 1, **characterized in that** valves or control means of the actuator unit (7) are arranged in front of and/or behind a piston/cylinder arrangement in the direction of walking.

3. The knee orthosis as claimed in claim 1 or 2, **characterized in that** the actuator unit (7) is arranged laterally alongside the thigh or lower leg and/or behind the structures (2, 3) in the direction of walking.

4. The knee orthosis as claimed in one of the preceding claims, **characterized in that** the actuator unit (7) is mounted, at least at one fastening point (73), via a bearing with at least two degrees of freedom.

5. The knee orthosis as claimed in claim 4, **characterized in that** the actuator unit (7) is mounted, at least at one fastening point, via a cardan bearing with intersecting axes, non-intersecting axes, or a ball-joint bearing.

6. The knee orthosis as claimed in one of the preceding claims, **characterized in that** the actuator unit (7) is arranged tilted in the lateral plane about an angle θ.

7. The knee orthosis as claimed in one of the preceding claims, **characterized in that** the foot part (4) has a dorsal spring (5) on which or in which at least one sensor (50) is arranged for detecting the forces acting in the spring (5), in particular the ankle moment.

8. The knee orthosis as claimed in one of the preceding claims, **characterized in that** a knee moment sensor and/or a knee angle sensor are arranged on the orthosis.

9. The knee orthosis as claimed in one of the preceding claims, **characterized in that** an absolute angle sensor for detecting the spatial orientation of at least one of the structures (2, 3) is mounted on the orthosis.

10. The knee orthosis as claimed in one of the preceding claims, **characterized in that** a control means (8) is provided for changing the behavior of the actuator unit (7), in particular the damping resistance, as a function of sensor signals.

11. The knee orthosis as claimed in one of the preceding claims, **characterized in that** the movement axis of the joint mechanism (6) is fixed by centers of two ball joints.

12. The knee orthosis as claimed in one of the preceding claims, **characterized in that** the thigh structure (2) and the lower leg structure (3) are connected to each other via a cardan joint or ball joint.

## Revendications

1. Orthèse de genou comprenant une structure crurale (2), en particulier attelle crurale, présentant un dispositif de fixation (21) pour la fixation à la cuisse, une structure pour la jambe inférieure (3), en particulier attelle pour la jambe inférieure, couplée de façon orientable par l'intermédiaire d'un dispositif d'articulation (6) à la structure crurale (2) et présentant un dispositif de fixation (31) pour la fixation à la jambe inférieure, et une unité d'actionnement (7) entre la structure crurale (2) et la structure pour la jambe inférieure (3), les points de fixation (72, 73) de l'unité d'actionnement (7) sur les structures crurale et pour la jambe inférieure (2, 3) et le centre de rotation du dispositif d'articulation (6) formant un triangle, et les points de fixation (72, 73) étant agencés sur les structures (2, 3) de façon que la ligne de liaison entre les points de fixation soit perpendiculaire à une ligne de fixation (IH) entre un point de fixation (73) et le centre de rotation (6), dans une position angulaire du genou sur une plage d'une flexion de la jambe inférieure par rapport à la cuisse comprise entre 0° et 90°, **caractérisée en ce que** l'unité d'actionnement (7) et/ou les points de fixation (72, 73) de l'unité d'actionnement (7) sont montés déplaçables ou orientables dans le plan latéral.

2. Orthèse de genou selon la revendication 1, **caractérisée en ce que** des clapets ou des dispositifs de pilotage de l'unité d'actionnement (7) sont agencés dans le sens de la marche devant et/ou derrière un assemblage piston et cylindre.

3. Orthèse de genou selon la revendication 1 ou 2, **caractérisée en ce que** l'unité d'actionnement (7) est agencée latéralement près de la cuisse ou de la jambe inférieure et/ou dans le sens de la marche derrière les structures (2, 3).

4. Orthèse de genou selon l'une quelconque des revendications précédentes, **caractérisée en ce qu'**en au moins un point de fixation (73) l'unité d'actionnement (7) est supportée par l'intermédiaire d'un système de palier à au moins deux degrés de liberté.

5. Orthèse de genou selon la revendication 4, **caractérisée en ce qu'**en au moins un point de fixation l'unité d'actionnement (7) est supportée par l'intermédiaire d'une articulation à cardan avec des axes se croisant, ne se croisant pas, ou un palier à rotule.

6. Orthèse de genou selon l'une quelconque des revendications précédentes, **caractérisée en ce que** l'unité d'actionnement (7) est agencée inclinée d'un angle Θ dans le plan latéral.

7. Orthèse de genou selon l'une quelconque des revendications précédentes, **caractérisée en ce que** la partie pied (4) présente un ressort dorsal (5) sur ou dans lequel est agencé au moins un capteur (50) pour relever les forces agissantes dans le ressort (5), en particulier celle du moment de force dans la cheville.

8. Orthèse de genou selon l'une quelconque des revendications précédentes, **caractérisée en ce qu'**un capteur de moment de force dans le genou et/ou un capteur d'angle du genou sont agencés sur l'orthèse.

9. Orthèse de genou selon l'une quelconque des revendications précédentes, **caractérisée en ce qu'**un capteur d'angle absolu est monté sur l'orthèse pour relever l'orientation d'au moins une des structures (2, 3) dans l'espace.

10. Orthèse de genou selon l'une quelconque des revendications précédentes, **caractérisée en ce qu'**un dispositif de pilotage est prévu pour modifier le comportement de l'unité d'actionnement (7), en particulier la résistance d'amortissement en fonction de signaux de capteur.

11. Orthèse de genou selon l'une quelconque des revendications précédentes, **caractérisée en ce que** l'axe de mouvement du dispositif d'articulation (6) est défini par des points centraux de deux articulations rotule.

12. Orthèse de genou selon l'une quelconque des revendications précédentes, **caractérisée en ce que** la structure crurale (2) et la structure pour la jambe inférieure (3) sont reliées par l'intermédiaire d'une articulation rotule ou à cardan.
